# FASCICULE DE BREVET EUROPEEN

(11) **EP 0 986 312 B1**
(45) Date de publication et mention de la délivrance du brevet: **17.01.2007**
(21) Numéro de dépôt: 98930751.7
(22) Date de dépôt: 22.05.1998
(51) Int. Cl.: A23J 1/20, A23C 9/146, C07K 14/47, A61K 8/00

(54) **PROCEDE DE TRAITEMENT D'UNE MATIERE PREMIERE LACTIQUE CONTENANT DU GMP**
VERFAHREN ZUR BEHANDLUNG EINES GMP ENTHALTENDEN MILCHPRODUKTS
METHOD FOR TREATING A LACTIC RAW MATERIAL CONTAINING GMP

(30) Priorité: 27.05.1997 EP 97201607
(43) Date de publication de la demande: 22.03.2000
(73) Titulaire: SOCIETE DES PRODUITS NESTLE S.A., 1800 Vevey (CH)
(72) Inventeur: ERDMANN, Peter, CH-3006 Bern (CH); NEUMANN, Fred, CH-3612 Steffisburg (CH)
(74) Mandataire: Dixon, Sarah
(86) Numéro de dépôt international: PCT/EP1998/003176
(87) Numéro de publication internationale: WO 1998/053702

(56) Documents cités:
- EP-A- 0 291 264
- EP-A- 0 397 571
- DE-A- 4 344 342
- GB-A- 2 188 526
- US-A- 5 280 107
- DATABASE WPI Section Ch, Week 9529 Derwent Publications Ltd., London, GB; Class A97, AN 95-220083 XP002044766 & JP 07 132049 A (SHOKUHIN SANGYO HIGH SEPARATION SYSTEM), 23 mai 1995 cité dans la demande
- PATENT ABSTRACTS OF JAPAN vol. 018, no. 032 (C-1154), 18 janvier 1994 & JP 05 262793 A (SNOW BRAND MILK PROD CO LTD), 12 octobre 1993
- OUTINEN M ET AL: "CHROMATOGRAPHIC ISOLATION OF -CASEIN MACROPEPTIDE FROM CHEESE WHEY WITH A STROING BASIC ANION EXCHANGE RESIN" MILCHWISSENSCHAFT, vol. 50, no. 10, 1 janvier 1995, pages 570-574, XP000539295
- S. MARCHALL: "Casein macropeptide from whey- A new product opportunity" FOOD RESEARCH QUARTERLY, vol. 51, no. 1, 1991, pages 86-91, XP002044765
- G. SMITHERS: "New casein products for the food industry" FOOD AUSTRALIA, vol. 43, no. 6, 1991, pages 252-254, XP002080868

## Description

L'invention a trait à un procédé de traitement d'une matière première lactique contenant le glycomacropeptide ou caséinoglycomacropeptide (ci-après GMP), dans le but d'en séparer le dit GMP.

Le GMP est un macropeptide phosphorylé et partiellement sialylé qui est formé par l'action d'une protéase, par exemple la présure sur la kappa-caséine du lait des mammifères. Il représente environ 20 % en poids des protéines du lactosérum doux obtenu après séparation de la caséine lors de la fabrication de fromages.

On connait un procédé de fabrication de GMP au niveau du laboratoire consistant à traiter une matière première d'origine lactique, telle que par exemple une caséine acide ou un caséinate, hydrolysés par la présure ou encore un lactosérum doux de fromagerie déminéralisé et délactosé, par l'acide trichloracétique de manière à précipiter les protéines, puis à recueillir le surnageant, à le dialyser et enfin à sécher le dialysat. Un tel procédé n'est pas industriel.

Un procédé de production de GMP à l'échelle industrielle, décrit dans EP-A- 0488589 consiste à traiter un produit lactosérique par échange d'ions, à recueillir la fraction n'ayant pas été adsorbée, à la concentrer et à la déminéraliser par ultrafiltration, diafiltration et le cas échéant osmose inverse et à recueillir le GMP.

Un procédé de production d'une fraction protéique de lactosérum est décrit dans UK-A-2188526. Celui-ci consiste à traiter un produit laitier par une résine anionique forte, dans des conditions telles que les protéines et certains peptides de la matière traitée sont adsorbés sur la résine de manière non-sélective sous forme de complexes. De tels complexes sont difficiles à éluer ensuite de la résine. L'éluat se caractérise par la formation d'un gel ferme à pH inférieur à 4,5 et à la température ambiante une fois mis en suspension dans l'eau. La fraction protéique peut être utilisée dans des boissons du genre des "milk-shake" et dans des mousses-desserts. EP-A-0397571 divulgue l'utilisation du caséinoglycopeptide k pour la fabrication d'un médicament pour la prévention et le traitement des thromboses.

Dans JP-A-07132049, on propose d'utiliser une résine d'échange d'ions anionique faible dont la matrice est hydrophile pour séparer les peptides sialylés du lactosérum. La méthode employée consiste à faire passer la matière première dont le pH a été préalablement réglé de manière précise, à une valeur de 4 à 6, sur un support macomoléculaire hydrophile consistant en un polysaccharide naturel ou un polyvinyle synthétique, greffé avec des groupes échangeurs basiques. Les supports utilisés comme matrice ne sont pas aisément applicables industriellement.

Le but de l'invention est la séparation sélective du GMP des autres composants des matières lactiques en une simple opération à l'échelle industrielle avec un rendement élevé.

L'invention concerne donc un procédé de traitement par échange d'ions d'une matière première lactique liquide contenant du GMP, dans le but de recueillir d'une part un produit utilisable directement comme source de protéine et d'autre part le GMP sous forme purifiée, caractérisé par le fait qu'il comprend les étapes suivantes:
i) Décationisation de la matière première liquide, de sorte que le pH ait une valeur de 1 à 4,5,
ii) Mise en contact du dit liquide avec une résine anionique faible de matrice hydrophobe, de manière prédominante sous forme alcaline jusqu'à un pH stabilisé,
iii) Séparation de la résine et du produit liquide que l'on recueille et
iv) Désorption du GMP de la résine.

Comme matière première lactique, on peut utiliser dans le procédé selon l'invention tout produit ou sous-produit contenant le GMP. On peut citer à titre indicatif:
- le lactosérum doux obtenu après séparation de la caséine coagulée par la présure,
- un lactosérum doux ou un tel lactosérum plus ou moins déminéralisé par exemple par électrodialyse, échange d'ions, osmose inverse, électrodéionisatiôn ou une combinaison de ces procédés,
- un concentrat de lactosérum doux,
- un concentrat de lactosérum doux plus ou moins déminéralisé par exemple par électrodialyse, échange d'ions, osmose inverse, électrodéionisation ou une combinaison de ces procédés,
- un concentrat de protéines de lactosérum doux fortement délactosé obtenu par exemple par ultrafiltration, puis diafiltration (ultrafiltration avec lavage),
- les eaux-mères de cristallisation du lactose à partir d'un lactosérum doux,
- un perméat d'ultrafiltration d'un lactosérum doux,
- le produit de l'hydrolyse par une protéase d'une caséine native obtenue par précipitation acide du lait écrémé par un acide minéral ou par acidification biologique, le cas échéant avec addition d'ions calcium ou encore d'une caséine micellaire, obtenue par exemple par microfiltration d'un lait écrémé,
- le produit de l'hydrolyse d'un caséinate par une protéase.

Une matière première préférée est un lactosérum doux de fromagerie préconcentré, de préférence à 10-23 % en poids et décationisé ou complétement déionisé, c'est à dire décationé et désanioné.

Une autre matière de choix de choix est un concentrat de protéines de lactosérum doux délactosé et décationé.

Ces matières premières peuvent se présenter sous forme liquide ou sous forme de poudre et dans ce dernier cas on les met en dispersion dans l'eau, de préférence déminéralisée en vue de leur traitement ultérieur.

Ces matières premières peuvent provenir de lait de ruminant tel que vache chèvre, brebis ou bufflesse.

Selon un premier mode de réalisation du procédé, on met la matière première liquide en présence de résine anionique faible dans un réacteur sous agitation modérée à une température < 50° C, de préférence comprise entre 0 et 15° C. L'agitation doit être juste nécessaire à la fluidisation du lit de résine. Celle-ci peut être produite par exemple par un agitateur ou, de préférence par l'introduction d'un courant de fluide, par exemple d'air ou d'azote sous pression par le bas du réacteur.

On peut utiliser toute résine échangeuse d'anions dont la matrice est hydrophobe et dont les groupes d'échange sont faiblement basiques sous forme de gel, macroporeuse ou macroréticulée, de préférence polystyrénique ou polyacrylique, particulièrement à base de copolymère polystyrène divinylbenzène et de préférence macroréticulée pour des raisons de résistance aux chocs osmotiques. Les groupes actifs sont généralement des amines de primaire à tertiaire. Une telle résine doit être de façon prédominante sous forme alcaline (qualifiée ci-après de forme OH⁻) et donc ses sites actifs doivent avoir été régénérés de préférence en grande partie sous cette forme.

Pendant cette mise en contact, les sites actifs de la résine sont échangés contre des molécules de GMP, ce qui produit une augmentation progressive du pH du liquide traité, jusqu'à une valeur finale stabilisée, par exemple de 4,5 à 5,5 selon la matière première mise en oeuvre. La durée de l'opération et les quantités respectives de résine et de liquide traité sont choisies en fonction de la composition du produit de départ et de la quantité de GMP désirée. Cette opération dure de 1 à 10 h, par exemple 4 h. Les proportions respectives de résine et de liquide à traiter peuvent varier grandement et sont, en volume de 1:1 à 1:30 et de préférence de 1:1 à 1:10, selon le degré souhaité de séparation du GMP.

Selon un autre mode de réalisation, on peut percoler le liquide dans une colonne remplie de la résine, en recueillir le liquide traité et récupérer le GMP adsorbé sur la résine par élution. On peut pour ce faire procéder en continu ou en semi-continu, par exemple en extrayant la résine saturée de la colonne à contre-courant et en la remplaçant par de la résine fraîchement régénérée.

On peut combiner les modes de réalisation précédents, en réacteur et en colonne, par exemple en utilisant un dispositif mixte dont la partie supérieure est un réacteur muni de moyens d'agitation ou de production d'un lit fluidisé contenant la résine, séparé par une grille ou un filtre d'une partie inférieure constituée d'une colonne où l'on peut réaliser en fin de traitement la récupération de la résine, par exemple par décantation, le soutirage du liquide traité.

Le liquide ainsi traité peut être concentré, par exemple par évaporation, puis séché, par exemple par pulvérisation dans une tour de séchage.

La poudre ainsi obtenue sert avantageusement de matière première protéique dans la préparation des produits infantiles et est remarquable du fait de son profil en acides aminés souhaités, son aminogramme montrant un appauvrissement en thréonine et un enrichissement en acides aminés aromatiques, tels que le tryptophane.

Pour en séparer le GMP, on traite d'abord la résine par lavage, par exemple avec de l'eau déminéralisée, puis le cas échéant avec une solution saline diluée ou une solution acide diluée et on la rince à l'eau déminéralisée. La désorption proprement dite du GMP a lieu avec une solution aqueuse d'acide, de base ou de sel, de préférence avec une solution aqueuse basique, par exemple de NaOH, de KOH ou de Ca(OH)₂, avantageusement de concentration < 8 % en poids, de préférence de 0,5 à 3 %, suivie d'un lavage à l'eau déminéralisée. De cette manière, la résine est régénérée par la même occasion. On joint ensuite l'éluat et les eaux de lavage, puis on les déminéralise, par exemple par ultrafiltration ou nanofiltration sur membrane de zone de coupure moyenne 3000 dalton et on sèche le rétentat, par exemple par lyophilisation.

Le GMP ainsi obtenu est sensiblement exempt de matière grasse et de lactose et pauvre en protéines de lactosérum.
Il contient de préférence, en poids:
< 1 % de matière grasse,
< 0,2 % de lactose et
< 3 % de protéines vraies de lactosérum.

Le GMP peut être utilisé dans ses applications connues, par exemple pour ses propriétés biologiques dans des compositions pharmaceutiques orales, parentérales ou sous-cutanées comme agent anti-diarrhéique ou anti-bactérien ou comme agent anti-plaque et anti-carie dans des compositions d'hygiène dentaire.

Un avantage non négligeable du procédé selon l'invention est qu'il n'y a pas de diminution de la performance de la résine ni d'encrassement de celle-ci, même après jusqu'à 150 cycles de traitement.

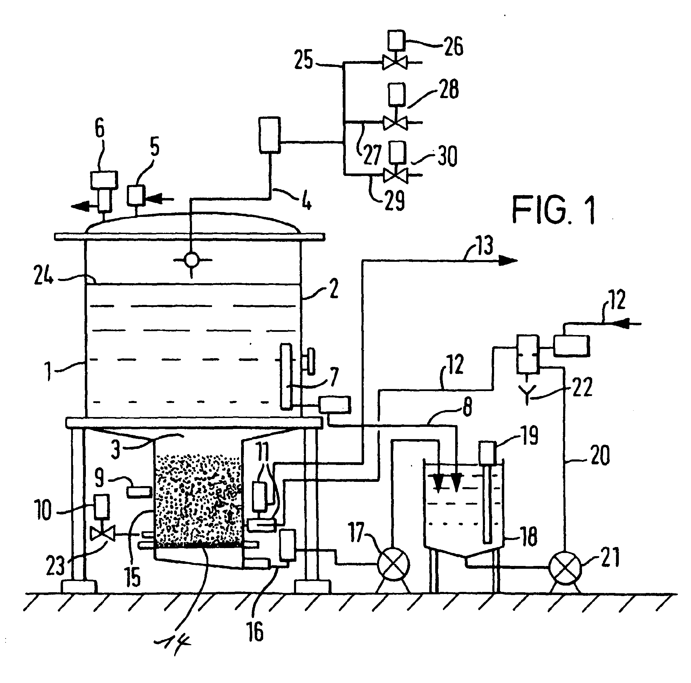

Les exemples ci-après illustrent l'invention, ainsi que la figure 1 du dessin, montrant, de manière schématique et à titre non-limitatif, un dispositif préféré de mise en oeuvre. Dans les exemples, les parties et pourcentages sont pondéraux sauf indication contraire.

### Exemple 1

Pour le traitement, on utilise le réacteur 1 constitué dans sa partie supérieure d'une cuve principale 2 communiquant dans sa partie inférieure avec un compartiment 3 de diamètre plus faible que celui de la cuve 2. La cuve 2 est pourvue d'un conduit d'amenée de liquide de rinçage 4, d'une amenée de gaz sous pression 5, d'une soupape de sécurité 6 permettant de réguler la pression de gaz dans le réacteur 1. A un niveau voisin de sa base la cuve 2 est pourvue d'une crépine 7 et d'un conduit de soutirage de liquide 8.

Au niveau du compartiment 3, le réacteur est muni d'un pH-mètre 9, d'une amenée de gaz 10 et communique par une vanne à trois voies 11 avec un conduit 12 d'amenée de liquide à traiter et un conduit d'évacuation 13 pour le liquide traité. A la base le compartiment 3 est pourvu d'une grille ou d'une plaque perforée 14 dont le rôle est de recueillir les billes de résine 15. En dessous de la grille 14, un conduit 16 de soutirage amène le liquide par l'intermédiaire de la pompe 17 vers la cuve tampon 18 munie d'un dispositif de contrôle de niveau 19 et delà vers le conduit 20 par l'intermédiaire de la pompe 21. Le conduit 20 est relié soit au conduit 12, soit au trop-plein d'évacuation 22.

On disperse un concentrat de protéines de lactosérum doux bovin, traité de manière classique par électrodialyse et décationé sur résine cationique forte, dans de l'eau déionisée de sorte que la solution ait une teneur en matière sèche de 6,5 %.

Le concentrat a la composition ci-après:

| | % |
|---|---|
| Protéines (GMP inclus) | 76 |
| Lactose | 4,8 |
| Cendres | 2,5 |
| Lipides | 8 |
| Eau | complément à 100 |

Par le conduit 12, on transfère 127 kg de la dispersion, de pH initial 4,25, à la température de 12° C dans le réacteur 1 par la base duquel on introduit de l'air en barbottage au niveau du compartiment 3, par l'amenée 10 par l'intermédiaire d'une vanne de non retour 23, de manière à créer un lit fluidisé de billes de résine 15 comprenant 23 kg de résine anionique faible de matrice hydrophobe à base de polystyrène (IMAC HP 661^{(R)}, Rohm & Haas, régénérée sous forme OH⁻). Les billes de résine 15 sont agitées pendant 4 h au contact de la dispersion du fait de la turbulence créée par la fluidisation. On contrôle constamment le pH au moyen du pH-mètre 9. La stabilisation du pH à 5,08 indique la fin de la réaction. On coupe alors l'arrivée d'air en 10 et on introduit de l'air par le haut du réacteur en 5 au dessus du niveau 24 de liquide, ce qui a pour effet de pousser le liquide et de décanter les billes de résine dans la partie inférieure 3 du réacteur 2 où elles sont retenues par la grille 14. On soutire le liquide traité par gravité par le conduit 8 et par le conduit 16 par l'intermédiaire de la pompe 17 vers la cuve tampon 18 et on l'évacue par le conduit 20 au moyen de la pompe 21 et delà vers la sortie par les conduits 12 et 13.

Après concentration du liquide à 28 % de matières sèches par évaporation, on sèche le concentrat par pulvérisation dans une tour de séchage (ces opérations n'étant pas représentées).

Une analyse du concentrat par chromatographie liquide à haute performance (HPLC) montre que la réaction a enlevé 91 % du GMP de départ. Par ailleurs, la poudre contient 95 % des protéines de lactosérum de départ.

Pour recueillir le GMP, on lave le réacteur et la résine avec de l'eau déionisée depuis le conduit 25 et la vanne 26, puis le conduit 4 à travers le réacteur jusqu'à la sortie par les conduits 12 et 13. Par le même circuit, on élue le GMP avec deux fois 40 l de solution aqueuse à 2 % de NaOH distribuée par le conduit 27 et la vanne 28 et on rince avec 30 l d'eau déionisée. Après avoir joint les volumes d'éluat et de rinçage, on concentre l'ensemble à un volume de 25 l par ultrafiltration ou nanofiltration avec une membrane de coupure nominale de 3.000 dalton, puis l'on sèche le rétentat par lyophilisation (ces opérations n'étant pas représentées) et l'on obtient 750 g de GMP, ce qui correspond à un rendement de 82 % par rapport au GMP de départ.

Périodiquement, la résine subit une régénération acide après la régénération alcaline une fois que l'on a traité l'équivalent de 10 volumes de lit de résine. Pour ce faire, après l'élution du GMP par la solution alcaline tel que décrit précédemment, on amène une solution aqueuse concentrée de HCl par le conduit 29 et la vanne 30, respectivement 25 pour l'eau. La résine est ensuite mise sous forme OH⁻ par passage d'une solution aqueuse concentrée de NaOH depuis les conduits 27, respectivement 25 pour l'eau, puis 4, et sort ensuite du réacteur 1 par le conduit 16, est repris par la pompe 17 vers la cuve tampon 18, puis par la pompe 21 et évacué par le conduit 20 et le trop-plein 22 vers le traitement des effluents. A la suite de cette opération, la résine est prête pour un autre cycle de traitement.

### Exemple 2

On utilise un lactosérum doux bovin, préalablement concentré à 17 % de matière sèche, puis déminéralisé par électrodialyse, décationé sur colonne de résine cationique forte, désanioné sur colonne de résine anionique faible et séché par pulvérisation en tour de séchage, de composition indiquée ci-après:

| | % |
|---|---|
| Protéines (GMP inclus) | 11,7 |
| Lactose | 81,7 |
| Cendres | 1 |
| Lipides | 1 |
| Eau | complément à 100 |

On solubilise cette poudre de lactosérum déminéralisée dans de l'eau déionisée. Après décationation, la solution a un pH initial de 3,8. Dans l'installation précédente, on traite 392 kg de cette solution à la température de 8° C en l'agitant dans le réacteur en présence de 23 kg de résine anionique faible de matrice hydrophobe à base de polystyrène (IMAC HP 661^{(R)}, Rohm & Haas, régénérée sous forme OH⁻) pendant 4 h. La stabilisation du pH à 4,89 indique la fin de la réaction. On soutire alors le liquide et on recueille la résine comme précédemment.

Après concentration du liquide à 45 % de matières sèches par évaporation, on sèche le concentrat par pulvérisation dans une tour de séchage.

Une analyse du concentrat par HPLC montre que la réaction a enlevé 89 % du GMP de départ. Par ailleurs, la poudre contient 9,1 % de protéines de lactosérum, ce qui correspond à un rendement de 90 % des protéines de lactosérum.

Pour recueillir le GMP, on lave la résine successivement avec de l'eau déionisée, avec 301 d'une solution aqueuse à 0,5 % de HCl et avec 301 d'eau déionisée, puis on élue le GMP avec deux fois 40 1 de solution aqueuse à 2 % de Ca(OH)₂ et on rince avec 30 1 d'eau désionisée. Après avoir joint les volumes d'éluat et de rinçage, on concentre l'ensemble à un volume de 25 1 par ultrafiltration avec une membrane de coupure nominale de 3.000 dalton, puis l'on sèche le rétentat par lyophilisation et l'on obtient 900 g de GMP, ce qui correspond à un rendement de 80 % par rapport au GMP de départ.

### Exemple 3

On part d'un lactosérum doux, préconcentré à 18 % de matière sèche, décationé par traitement sur une colonne de résine cationique forte, dont le pH initial est 1,09.

Dans l'installation précédente, on traite 70 kg de ce lactosérum à la température de 25° C en l'agitant dans le réacteur en présence de 14 kg de résine anionique faible de matrice hydrophobe à base de polystyrène (IRA 96^{(R)}, Rohm & Haas, régénérée sous forme OH⁻) pendant 4 h. L'agitation se fait par création d'un lit fluidisé de billes de résine avec barbottage d'azote. La stabilisation du pH à 4,79 indique la fin de la réaction. On sépare alors le liquide de la résine comme précédemment. Après concentration du liquide à 45 % de matières sèches par évaporation, on sèche le concentrat par pulvérisation dans une tour de séchage.

Une analyse de la poudre par HPLC montre que la réaction a enlevé 85 % du GMP de départ. Par ailleurs, la poudre contient 9,2 % des protéines de lactosérum, ce qui correspond à un rendement de 90 % des protéines de lactosérum.

L'analyse de l'aminogramme du concentrat montre un profil se caractérisant par une diminution de 28 % de la thréonine, par une augmentation de 18 % de l'arginine et par une augmentation de 20 % du tryptophane.

Pour recueillir le GMP, on lave la résine successivement avec de l'eau déionisée, avec 50 1 d'une solution aqueuse à 0,05 % de NaCl et deux fois 50 l d'eau déionisée, puis on élue le GMP avec deux fois 25 l de solution aqueuse à 0,6 % de KOH et on rince avec 10 l d'eau déionisée. Après avoir joint les volumes d'éluat et de rinçage, on concentre l'ensemble à un volume de 25 1 par ultrafiltration avec une membrane de coupure nominale de 3.000 dalton, puis l'on sèche le rétentat par lyophilisation et l'on obtient 175 g de GMP, ce qui correspond à un rendement de 80 % par rapport au GMP de départ.

### Exemple 4

On part d'une poudre de perméat d'ultrafiltration de lactosérum doux débarrassée de la plupart de ses sels dont la composition est la suivante:

| | % |
|---|---|
| Protéines (GMP inclus) | 2,75 |
| Lactose | > 90 |
| Cendres | 1,5 |
| Eau | complément à 100 |

On dissout la poudre précédente dans l'eau déminéralisée, de sorte que la solution ait une teneur en matières sèches de 19,35 %. On décationne cette solution par passage sur une colonne de résine cationique forte IR 120^{(R)} (Rohm & Haas), ce qui conduit à une solution contenant 18,73 % de matières sèches dont le pH est 2,77.

On agite 565 g de cette solution et 56,5 g de résine anionique faible de matrice hydrophobe à base de polystyrène (IMAC HP 661^{(R)}, Rohm & Haas, régénérée sous forme OH⁻) pendant 3 h à 10° C jusqu'à stabilisation du pH à la valeur finale 4,53. On sépare ensuite le perméat ainsi traité des billes de résine par filtration et on le sèche par lyophilisation.

Le perméat de protéines de lactosérum ainsi traité contient 1,75 % de protéines. L'analyse de son aminogramme montre un profil se caractérisant par une diminution de 20 % de la thréonine et par une augmentation de 50 % du tryptophane.

Pour recueillir le GMP, on lave la résine avec 11 d'eau déionisée, puis on élue le GMP avec 50 ml de solution aqueuse à 0,6 % de NaOH, puis on rince avec 20 ml d'eau déionisée. Après avoir joint les volumes d'éluat et de rinçage, on concentre l'ensemble par ultrafiltration avec une membrane de coupure nominale de 3.000 dalton, puis l'on sèche le rétentat par lyophilisation et l'on obtient 870 mg de GMP.

### Exemple 5

On percole 3,5 1 de lactosérum doux préconcentré à 20 % de matière sèche, décationé sur colonne de résine cationique forte et de pH 1,09 à travers une colonne contenant 450 ml de résine anionique faible de matrice hydrophobe à base de polystyrène (IMAC HP 661^{(R)}, Rohm & Haas), à raison de 2 volumes de lit/h.

On recueille l'équivalent de 4 volumes de lit, constituant 4 fractions égales de pH allant de 6 à 3 et dont la quantité de GMP enlevé va de 50 à 9 % (évalué par HPLC). Après réunion des 4 fractions, on obtient une solution de pH 4,5 dans laquelle 25 % du GPM a été enlevé (par rapport à la matière lactosérique de départ).

Pour recueillir le GMP, on procède comme à l'exemple 1 et on obtient des résultats équivalents quant à la pureté du GMP.

## Revendications

1. Procédé de traitement par échange d'ions d'une matière première liquide lactique contenant du GMP, dans le but de recueillir d'une part un produit utilisable comme source de protéine et d'autre part le GMP sous forme purifiée, **caractérisé par le fait qu'**il comprend les étapes suivantes:
i) Décationisation de la matière première liquide, de sorte que le pH ait une valeur de 1 à 4,5,
ii) Mise en contact du dit liquide avec une résine anionique faible de matrice hydrophobe, de manière prédominante sous forme alcaline jusqu'à un pH stabilisé,
iii) Séparation de la résine et du produit protéique liquide que l'on recueille et
iv) Désorption du GMP de la résine.

2. Procédé selon la revendication 1, **caractérisé par le fait que** la matière première est un lactosérum doux de fromagerie préconcentré, de préférence à 10-23 % en poids et décationé ou complétement déionisé.

3. Procédé selon la revendication 1, **caractérisé par le fait que** la matière première est un concentrat de protéines de lactosérum doux délactosé et décationé.

4. Procédé selon la revendication 1, **caractérisé par le fait que** la matière première est le produit de l'hydrolyse par une protéase d'une caséine native obtenue par précipitation acide du lait écrémé par un acide minéral ou par acidification biologique, le cas échéant avec addition d'ions calcium, le produit de l'hydrolyse par une protéase d'une caséine micellaire, obtenue par exemple par microfiltration d'un lait écrémé ou encore le produit de l'hydrolyse d'un caséinate par une protéase.

5. Procédé selon la revendication 1, **caractérisé par le fait que** la matière première est un perméat d'ultrafiltration de lactosérum doux.

6. Procédé selon la revendication 1, **caractérisé par le fait que** l'on met la matière première liquide en présence de résine anionique faible de manière prédominante sous forme alcaline dans un réacteur sous agitation modérée à une température < 50° C, de préférence comprise entre 0 et 15° C, pendant une à plusieurs heures, ce qui produit une augmentation progressive du pH du liquide traité, jusqu'à stabilisation, puis que l'on sépare le liquide de la résine par filtration ou centrifugation.

7. Procédé selon la revendication 6, **caractérisé par le fait que** l'on utilise toute résine échangeuse d'anions faiblement basique sous forme de gel, macroporeuse ou macroréticulée, dont la matrice est hydrophobe.

8. Procédé selon la revendication 6, **caractérisé par le fait que** l'on concentre le liquide ainsi traité, notamment par évaporation, puis qu'on le sèche, notamment par pulvérisation dans une tour de séchage.

9. Procédé selon la revendication 1, **caractérisé par le fait que**, pour en séparer le GMP sous forme purifiée, on traite d'abord la résine par lavage, puis on désorbe le GMP avec une solution aqueuse acide, basique ou saline, notamment de NaOH, KOH ou Ca (OH)₂, de concentration < 8 %, on la rince à l'eau déminéralisée, on joint ensuite l'éluat et les eaux de rinçage, puis on les déminéralise, notamment par ultrafiltration ou nanofiltration sur membrane de zone de coupure moyenne environ 3000 dalton et que l'on sèche le rétentat, notamment par lyophilisation.

10. Utilisation du GMP susceptible d'être purifié obtenu par le procédé selon la revendication 9 pour préparer une composition pharmaceutique, anti-diarrhéique ou anti-bactérienne.

11. Utilisation du GMP susceptible d'être purifié obtenu par le procédé selon la revendication 9 pour préparer une composition d'hygiène dentaire anti-plaque et anti-carie.

## Claims

1. Process for the treatment by ion exchange of a liquid lactic raw material containing GMP, with the aim of collecting on the one hand a product that can be used as a protein source and on the other hand the GMP in purified form, **characterised in that** it comprises the following steps:
i) decationisation of the liquid raw material so that the pH has a value of 1 to 4.5,
ii) bringing the said liquid into contact with a weak anionic resin having a hydrophobic matrix, predominantly in alkaline form, until a stabilised pH is reached,
iii) separation of the resin and the liquid protein product, which is collected, and
iv) desorption of the GMP from the resin.

2. Process according to claim 1, **characterised in that** the raw material is a sweet whey from cheese-making, which has been preconcentrated, preferably to 10-23 wt.%, and decationised or completely deionised.

3. Process according to claim 1, **characterised in that** the raw material is a delactosed, decationised sweet whey protein concentrate.

4. Process according to claim 1, **characterised in that** the raw material is the product of hydrolysis by a protease of a native casein obtained by acid precipitation of skimmed milk by a mineral acid or by biological acidification, with the addition of calcium ions if necessary, the product of hydrolysis by a protease of a micellar casein, obtained e.g. by microfiltration of a skimmed milk, or the product of hydrolysis of a caseinate by a protease.

5. Process according to claim 1, **characterised in that** the raw material is a sweet whey ultrafiltration permeate.

6. Process according to claim 1, **characterised in that** the liquid raw material is brought into the presence of a weak anionic resin predominantly in alkaline form in a reactor with moderate agitation at a temperature of <50°C, preferably from 0 to 15°C, for one to several hours, which produces a gradual increase in the pH of the liquid being treated until this stabilises, and that the liquid is then separated from the resin by filtration or centrifugation.

7. Process according to claim 6, **characterised in that** any weakly basic anion exchange resin is used in the form of a macroporous or macrocrosslinked gel having a hydrophobic matrix.

8. Process according to claim 6, **characterised in that** the liquid treated in this way is concentrated, particularly by evaporation, and then dried, particularly by spraying in a drying tower.

9. Process according to claim 1, **characterised in that**, to separate the GMP in purified form therefrom, the resin is first treated by washing and then the GMP is desorbed with an acidic, basic or saline aqueous solution, particularly of NaOH, KOH or Ca(OH)₂, with a concentration of <8%, rinsed with demineralised water, the eluate and the rinsing waters are subsequently combined and then demineralised, particularly by ultrafiltration or nanofiltration through a membrane with an average cutoff range of about 3000 dalton, and the retentate is dried, particularly by freeze-drying.

10. Use of the GMP capable of being purified, obtained by the process according to claim 9, to prepare an antidiarrhoeal or antibacterial pharmaceutical preparation.

11. Use of the GMP capable of being purified, obtained by the process according to claim 9, to prepare an antiplaque and anticaries dental hygiene composition.

## Patentansprüche

1. Verfahren zur Behandlung eines GMP enthaltenden flüssigen Milchausgangsmaterials durch Ionenaustausch zur Gewinnung einerseits eines als Proteinquelle verwendbaren Produkts und andererseits von GMP in gereinigter Form, **dadurch gekennzeichnet, dass** es die folgenden Schritte umfasst:
i) Befreiung des flüssigen Ausgangsmaterials von Kationen so, dass der pH einen Wert von 1 bis 4,5 aufweist,
ii) In-Kontakt-Bringen dieser Flüssigkeit mit einem schwach anionischen Harz mit hydrophober Matrix überwiegend in alkalischer Form bis zu einem stabilisierten pH,
iii) Trennung des Harzes und des flüssigen Proteinprodukts, das man gewinnt, und
iv) Desorption des GMP von dem Harz.

2. Verfahren nach Anspruch 1, **dadurch gekennzeichnet, dass** das Ausgangsmaterial eine vorzugsweise auf 10-23 Gew.-% vorkonzentrierte und von Kationen oder vollständig von Ionen befreite Käserei-Süßmolke ist.

3. Verfahren nach Anspruch 1, **dadurch gekennzeichnet, dass** das Ausgangsmaterial ein von Lactose und von Kationen befreites Süßmolkeproteinkonzentrat ist.

4. Verfahren nach Anspruch 1, **dadurch gekennzeichnet, dass** das Ausgangsmaterial das Produkt der mit einer Protease durchgeführten Hydrolyse von nativem Casein, das durch saure Ausfällung von entrahmter Milch durch eine mineralische Säure oder durch biologische Ansäuerung erhalten wird, ggf. unter Zusatz von Calciumionen, das Produkt der mit einer Protease vorgenommenen Hydrolyse eines mizellären Caseins, das beispielsweise durch Mikrofiltration von entrahmter Milch erhalten wird, oder das Produkt der Hydrolyse eines Caseinats mit einer Protease ist.

5. Verfahren nach Anspruch 1, **dadurch gekennzeichnet, dass** das Ausgangsmaterial ein Süßmolke-Ultrafiltrationspermeat ist.

6. Verfahren nach Anspruch 1, **dadurch gekennzeichnet, dass** man das flüssige Ausgangsmaterial mit schwach anionischem Harz vorwiegend in alkalischer Form in einem Reaktor unter moderatem Rühren bei einer Temperatur <50°C, vorzugsweise zwischen 0 und 15°C, während einer oder mehrerer Stunden zusammenbringt, was eine allmähliche Erhöhung des pH-Werts der behandelten Flüssigkeit bis zur Stabilisierung bewirkt, und dass man dann die Flüssigkeit von dem Harz durch Filtration oder Zentrifugierung abtrennt.

7. Verfahren nach Anspruch 6, **dadurch gekennzeichnet, dass** man jedes beliebige makroporöse oder makrovernetzte schwach basische Anionenaustauschharz in Form von Gel, dessen Matrix hydrophob ist, verwendet.

8. Verfahren nach Anspruch 6, **dadurch gekennzeichnet, dass** man die auf diese Weise behandelte Flüssigkeit insbesondere durch Eindampfen behandelt und sie dann insbesondere durch Sprühtrocknung in einem Trockenturm trocknet.

9. Verfahren nach Anspruch 1, **dadurch gekennzeichnet, dass** man zunächst das Harz, um das GMP in gereinigter Form von ihm abzutrennen, durch Waschen behandelt, dann das GMP mit einer sauren, basischen oder salzhaltigen wässrigen Lösung, insbesondere NaOH, KOH oder Ca(OH)₂, mit einer Konzentration <8% desorbiert, es mit entmineralisiertem Wasser spült, dann das Eluat und das Spülwasser vereinigt, sie insbesondere durch Ultrafiltration oder Nanofiltration über eine Membran mit einer mittleren Schnittzone von etwa 3000 Dalton entmineralisiert und dass man das Retentat insbesondere durch Lyophilisierung trocknet.

10. Verwendung von GMP, das gereinigt werden kann und mit Hilfe des Verfahrens nach Anspruch 9 erhalten wird, für die Herstellung einer antidiarrhoeischen oder antibakteriellen pharmazeutischen Zusammensetzung.

11. Verwendung von GMP, das gereinigt werden kann und mit Hilfe des Verfahrens nach Anspruch 9 erhalten wird, für die Herstellung einer Zahnhygienezusammensetzung gegen Plaque und Karies.
